# EUROPEAN PATENT SPECIFICATION

(11) **EP 3 907 234 B1**
(45) Date of publication and mention of the grant of the patent: **29.10.2025**
(21) Application number: 19918805.3
(22) Date of filing: 26.06.2019
(51) Int. Cl.: C07K 5/02, C07K 5/062, C07K 5/027, C07K 1/06, A61K 47/68, A61K 47/65, C07K 1/113

(54) **PREPARATION METHOD FOR AND INTERMEDIATE OF DRUG-LINKER FOR ANTIBODY DRUG CONJUGATE MC-MMAF**
VERFAHREN ZUR HERSTELLUNG UND ZWISCHENPRODUKT EINES ARZNEIMITTEL-LINKERS FÜR EIN ANTIKÖRPER-ARZNEIMITTEL-KONJUGAT MC-MMAF
PROCÉDÉ DE PRÉPARATION ET INTERMÉDIAIRE DE MÉDICAMENT-LIEUR POUR UN CONJUGUÉ ANTICORPS-MÉDICAMENT MC-MMAF

(30) Priority: 08.03.2019 CN 201910178142
(43) Date of publication of application: 10.11.2021
(73) Proprietor: Levena Biopharma Co., Ltd., SIP Suzhou Jiangsu 215123 (CN)
(72) Inventor: XU, Zhe, Suzhou, Jiangsu 215123 (CN); LI, Haihong, Suzhou, Jiangsu 215123 (CN); GUO, Maojun, Suzhou, Jiangsu 215123 (CN); LI, Hui, Suzhou, Jiangsu 215123 (CN)
(74) Representative: Cleanthous, Marinos
(86) International application number: PCT/CN2019/092950
(87) International publication number: WO 2020/181687

(56) References cited:
- WO-A1-2019/042447
- WO-A2-2018/140275
- CN-A- 109 912 684

## Description

### TECHNICAL FIELD

The present disclosure relates to the field of organic synthesis, in particular to a method for preparing drug-linker MC-MMAF for antibody drug conjugates and intermediates therein.

### BACKGROUND

Antibody drug conjugate (ADC) is a new type of anti-tumor drug. Its principle is to connect cytotoxin to antibody. Through antibody recognition of specific antigen on the surface of cancer cell, it enters the cancer cells through endocytosis, thereby transporting cytotoxins to the target, achieve the purpose of targeted treatment of malignant tumors. Compared with traditional small-molecule anti-tumor drugs, ADC is more specific and effective because it can rely on the target recognition of antibodies and the high activity of toxins.

ADC includes three different components, namely antibody, linker and cytotoxin. The antibody achieves targeting, and the linker ensures the stability of the ADC during blood transport, and after reaching the target point, the toxin exerts a killing effect on cancer cells. According to different mechanisms of action, toxins appropriate for ADC are classified into microtubule inhibitors, DNA damaging agents, RNA polymerase inhibitors, and et al.. At present, the toxins used by ADCs commercial available and in clinical trials are mainly microtubule inhibitors, mainly including dolastatin-based compounds, such as MMAE, MMAF and MMAD, and maytansine-based (Maytansine-based) designed compounds, such as DM1 and DM4. In terms of linkers, the main applications are non-cleavable types, such as valine-citrulline (Valine-Citriline) and cyclohexyl carboxylic acid (MCC). After lysosomal hydrolysis, the drug is still active, and is connected to a certain amino acid residue through link area.

There are many ways to form antibody-drug conjugates. Either the amino or sulfhydryl group on the antibody and the drug linker can be chemically coupled, or the antibody can be modified. After a specific functional group is introduced on the antibody, it can be coupled with the drug linker for chemical reaction or enzyme-catalyzed reaction coupling. The structure of the drug-linker MC-MMAF involved in the present disclosure is shown below.

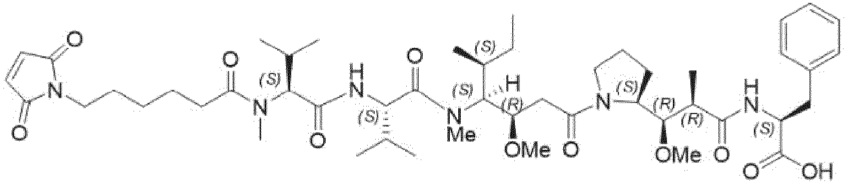

The synthesis route of MC-MMAF currently reported in the literature is to use the toxin MMAF and MC-hex-Acid (1-maleimido n-hexanoic acid) to perform a dehydration reaction to obtain MC-MMAF. The structure of MMAF is:

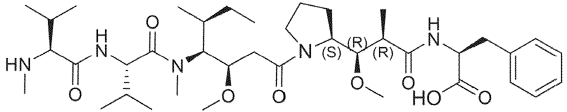

The synthesis scheme reported in the literature is:

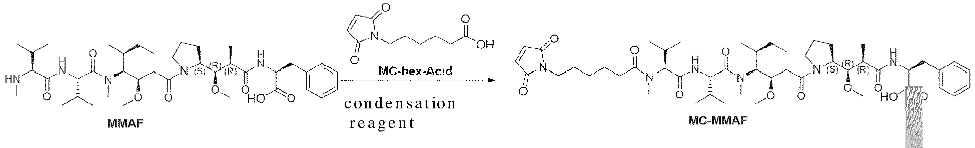

The N-terminal valine of this route of MMAF has a methyl group on the N, which is sterically hindered. In this case, the reaction speed of connecting 1-maleimido-n-hexanoic acid to MMAF will be slower. Even if a different amide condensing agent is used, it will cause racemization of the chiral carbon linked to the phenylpropionamide group of MMAF. This route is used for the synthesis of MC-MMAF of less than 1g, and finally high-pressure reverse phase preparation is used to remove isomeric impurities, and the yield is less than 50%.

This reaction route shows certain defects during scale-up production, such as: 1. Because the condensing agent will activate the carboxyl group on MMAF at the same time, this method will cause 30-50% racemization, forming difficult-to-remove isomer impurities, affecting yield; 2. Due to the aforementioned steric hindrance, the reaction time is long, and there are many impurities, which cause difficulties in the post-treatment and purification of the reaction; 3. The final product requires high-pressure reverse phase preparation to remove isomers, which increases operating costs; 4. Direct using the toxin MMAF as a raw material, it is necessary to do a good protection in scale-up of synthesis operations, and the selection of protective equipment will bring obstacles to the production operation.

WO2018140275 discloses a compound according to the general Formula (8): wherein R1 is hydrogen or C1-C4 alkyl; R2 is C1-C4 alkyl, benzyl, or 1H-indol-3-ylmethyl; T is selected from the group consisting of -CH(OR3)-R4, -C(=O)-OR3, or -C(=O)-NR3 R5, wherein R3 is hydrogen or C1-C4 alkyl; R4 is phenyl; R5 is hydrogen or C1-C4 alkyl; L1 is R6-C(=O)-R7; where R6 is C1-C6 alkyl; R7 is a bond or NH-NH; L2 is a bond,, or; where n is 1-4; and Ab is an antibody or antigen-binding antibody fragment, and a process for preparing the same. In certain embodiments, Ab is an antibody or antigen-binding antibody fragment which binds to C4.4a.

WO2019042447 discloses a novel toxin and a method for preparing an intermediate thereof. Specifically provided are a compound as represented by formula (III) and a method for preparing same, and a method for preparing a compound as represented by formula (I) which is obtained from the compound as represented by formula (III) by means of a series of protecting group introduction, protecting group removal, and condensation reactions.

### SUMMARY

On the one hand, in view of the defects in the prior art, the present disclosure provides a method for synthesizing MC-MMAF. The key to the reaction is to use a compound of structural formula 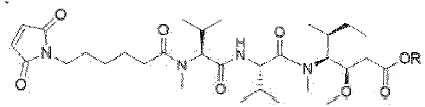 to condense with the structural fragment peptide Dap-Phe-OH of MMAF to directly obtain MC -MMAF or a salt thereof, R is selected from a group consisting of succinimidyl, p-nitrophenyl, phthalamide, and a mixture thereof.

The chemical structure of Dap-Phe-OH is as follows:

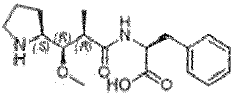

The above-mentioned objects of the present disclosure are achieved by the following technical solutions.

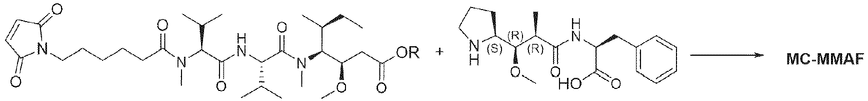

The synthesis method includes the following steps: 1) Dissolve the compound 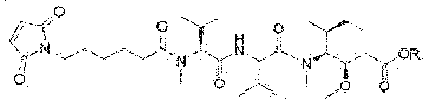 in an appropriate solvent and an amide condensation reaction occurs with Dap-Phe-OH to obtain MC-MMAF.

Preferably, in step 1), the appropriate solvent is selected from a group consisting of dichloromethane, dimethyl sulfoxide, N,N-dimethylformamide, N,N-dimethylacetamide, tetrahydrofuran, 1, 4-dioxane, 2-methyltetrahydrofuran and a mixture thereof; more preferably, the appropriate solvent is selected from a group consisting of dichloromethane and N,N-dimethylformamide and a mixture thereof.

Preferably, in step 1), if R is selected from a group consisting of p-nitrophenyl group, phthalamide group and a mixture thereof, in the presence of reagent P, it reacts with Dap-Phe-OH to obtain MC-MMAF. The reagent P is selected from a group consisting of triethylamine, diisopropylethylamine (DIEA), pyridine, N,N-dimethyl-4-pyridine, sodium carbonate, sodium bicarbonate, potassium carbonate, potassium bicarbonate, lithium carbonate, lithium bicarbonate and a mixture thereof, preferably sodium carbonate, or diisopropylethylamine (DIEA). The reaction temperature is 0°C to 100°C, preferably 15°C to 50°C.

Preferably, in step 1), it further comprises a step of separating MC-MMAF from the reaction liquid after the reaction is completed.

Preferably, the separation comprises evaporating the solvent under reduced pressure, and then purifying or recrystallizing by medium pressure chromatography to obtain MC-MMAF.

The preparation method of the present disclosure improves the reactivity of the N-terminal, thereby effectively controlling the occurrence of racemization; does not directly use the toxin MMAF, but uses fragmented peptides with lower toxicity, which minimizes the operational difficulty in scale-up production; no reverse phase preparation is required, it is easy to prepare and operate. As mentioned above, the method minimizes the difficulty of operation, makes the quality standard easier to control, and can be applied to the preparation of one hundred grams.

On the other hand, this patent also provides an intermediate compound for the synthesis of MC-MMAF, the structural formula 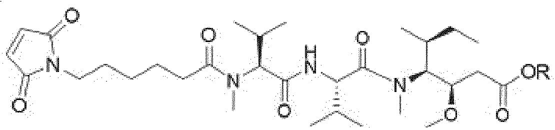 wherein R is selected from a group consisting of succinimidyl, p-nitrophenyl, phthalamide and a mixture thereof. It is preferably the following compound 2, as shown in Table 1:

**Table 1**

| No. | Structural formula |
|---|---|
| Compond 1 | 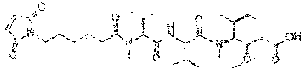 |
| Compoud 2 | 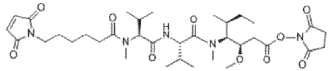 |

In another aspect, the present disclosure also provides a method of synthesis 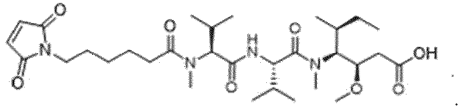 The synthesis steps are as follows:

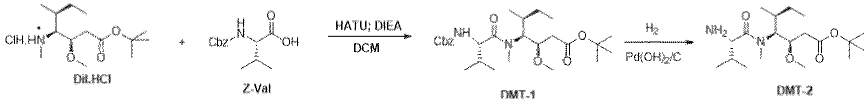

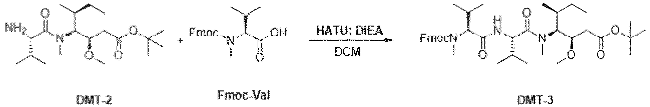

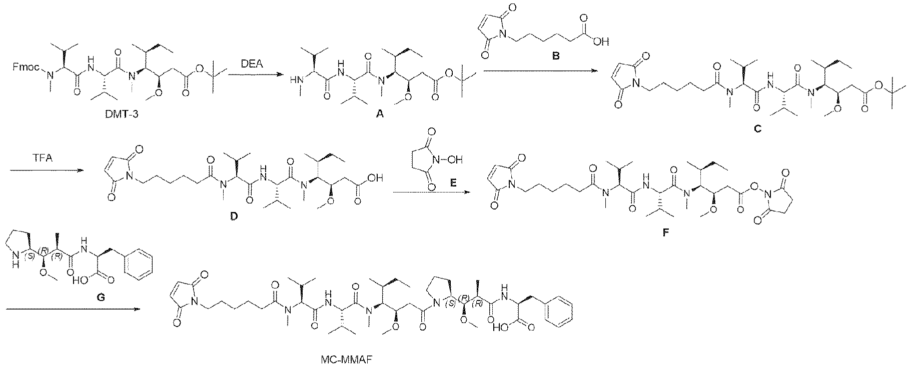

This route contains synthesizing of important intermediate D, and there is no report on the synthesis method of this compound before.

Compound D cannot be synthesized with polypeptide X without a protective group. In the experiment, a self-condensed product of polypeptide X was obtained. That is, the synthesis route shown below cannot directly synthesize compound D.

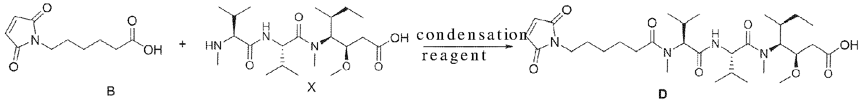

In the technical scheme employed in the present disclosure, compound C is first synthesized using a polypeptide with a protective group, and then compound D is obtained by deprotecting the protective group under acidic conditions. However, it was found in experiments that compound C and compound D are very unstable under acidic conditions, and the amide bond in the middle of the molecule will be broken, resulting in a very low yield. After further research, it is found that increasing the concentration of acid will greatly increase the speed of deprotection, but not much for the speed of side reactions. The concentration of trifluoroacetic acid (this concentration refers to the concentration of trifluoroacetic acid in the reaction solution in a solvent such as dichloromethane) ranges from 30% to 50%, preferably 35%, the deprotection reaction will be completed quickly and then quenched immediately. Finally, the yield of compound D can be increased from 5% to 50%.

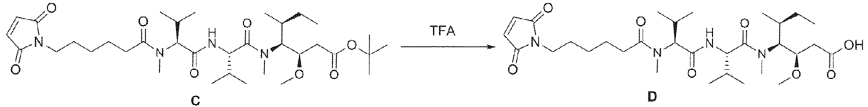

### side effects:

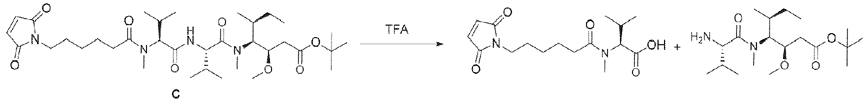

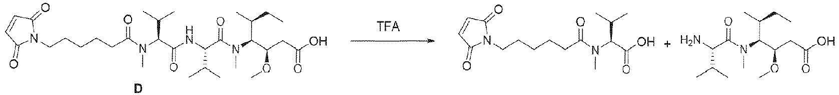

By selecting acid reagents and controlling the reaction conditions of acid concentration, the yield of compound D is greatly improved, making this route possible to be applied to production.

The present disclosure abandons the existing MMAF synthesis route and regards MC-MMAF as a whole to synthesize. The biggest problem is that MC linkers are fragments with relatively high reactivity. Connecting MC in advance will increase the difficulty of synthesis. Those skilled in the art would not think of this route. Through many studies, we have solved the problem of instability in the synthesis of the MC fragment compound introduced in advance, so that this overall synthetic route can be realized.

As used in this article, the definitions of commonly used organic abbreviations and their corresponding CAS numbers are shown in Table 2:

**Table 2**

| Abbreviat ion | Definition | CAS No. |
|---|---|---|
| BrOP | Bromotris(dimethylamino)phosphorus hexafluorophosphate | 50296-37-2 |
| DBU | 1,8-diazabicyclo[5.4.0]undec-7-ene | 6674-22-2 |
| DECP | Diethyl cyanophosphate | 2942-58-7 |
| DIEA | N,N-Diisopropylethylamine | 7087-68-5 |
| DMT | Dimethyl Val-Val-Dil-OH | 133120-89-5 |
| HOSu | N-hydroxysuccinimide | 6066-82-6 |
| TEA | Triethylamine | 121-44-8 |
| DCC | N,N'-Dicyclohexylcarbodiimide | 538-75-0 |
| EDCI | 1-(3-Dimethylaminopropyl)-3-ethylcarbodiimide hydrochloride | 7084-11-9 |
| DIC | N,N'-Diisopropylcarbodiimide | 693-13-0 |
| HATU | 2-(7-Azobenzotriazole)-N,N,N',N'-tetramethylurea hexafluorophosphate | 148893-10-1 |
| HBTU | Benzotriazole-N,N,N',N'-tetramethylurea hexafluorophosphate | 94790-37-1 |
| HBPIPU | (Benzotriazol-1-yloxy)dipiperidine carbohexafluorophosphate | 206752-41-2 |
| HBPyU | O-(benzotriazol-1-yl)-N,N,N',N'-dipyrrolylurea hexafluorophosphate | 105379-24-6 |
| HSPyU | Dipyrrolidinyl (N-succinimidyloxy) hexafluorophosphate | 207683-26-9 |
| HCTU | 6-Chlorobenzotriazole-1,1,3,3-tetramethylurea hexafluorophosphate | 330645-87-9 |
| HOTU | O-[(Ethoxycarbonyl)cyanomethylamine]-N,N,N',N'-tetramethylt hiourea hexafluorophosphate | 333717-40-1 |
| HOTT | N,N,N',N'-Tetramethyl-S-(1-oxo-2-pyridyl)thiourea hexafluorophosphate | 212333-72-7 |
| HSTU | N,N,N',N'-tetramethylurea-O-(N-succinimidyl) hexafluorophosphate | 265651-18-1 |
| HDMA | 1-[(Dimethylamino)(morpholine)methyl]-3-oxo-1H-[1,2,3]triazol e[4,5-b]pyridine 3-hexafluorophosphate | 958029-37-3 |
| TATU | 2-(7-Azabenzotriazole)-N,N,N',N'-tetramethylurea tetrafluoroborate | 873798-09-5 |
| TBTU | O-benzotriazole-N,N,N',N'-tetramethylurea tetrafluoroborate | 125700-67-6 |
| TCTU | O-(6-Chloro-1H-benzotriazol-1-yl)-N,N,N',N'-tetramethylurea tetrafluoroborate | 330641-16-2 |
| TCFH | N,N,N',N'-Tetramethylchloroformamidine hexafluorophosphate | 94790-35-9 |
| TDBTU | N,N,N',N'-tetramethyl-O-(4-carbonyl-3,4-dihydro-1,2,3-benzotri azin-3-yl)urea tetrafluoroborate | 125700-69-8 |
| TOTU | O-[(Ethoxycarbonyl)cyanomethylamine]-N,N,N',N'-tetramethylt hiourea tetrafluoroboron | 136849-72-4 |
| TOTT | 2-(1-Pyridin-2-yl oxide)-1,1,3,3-Tetramethylisothiourea tetrafluoroborate | 255825-38-8 |
| TPTU | 2-(2-pyridone-1-yl)-1,1,3,3-tetramethylurea tetrafluoroborate | 125700-71-2 |
| TFFH | Fluoro-N,N,N',N'-tetramethylurea hexafluorophosphate | 164298-23-1 |
| BTFFH | N,N,N',N'-bis(tetramethylene)fluoroformamidine hexafluorophosphate | 164298-25-3 |
| TNTU | 2-(Endo-5-norbornene-2,3-dicarboximide)-1,1,3,3-tetramethylure a tetrafluoroborate | 125700-73-4 |
| TSTU | 2-succinimidyl-1,1,3,3-tetramethylurea tetrafluoroborate | 105832-38-0 |
| COMU | cycluron | 2163-69-1 |
| T3P | Propyl phosphate tricyclic anhydride | 68957-94-8 |
| BOP | 1H-benzotriazol-1-yloxotris(dimethylamino)phosphonium hexafluorophosphate | 56602-33-6 |
| PyBOP | 1H-benzotriazol-1-yloxytripyrrolidinyl hexafluorophosphate | 128625-52-5 |
| PyBrOP | Tripyrrolidinylphosphonium bromide hexafluorophosphate | 132705-51-2 |
| PyClOP | Chlorotripyrrolidinyl hexafluorophosphate | 133894-48-1 |
| BrOP | Bromotris(dimethylamino)phosphonium hexafluorophosphate | 50296-37-2 |
| PyAOP | (3H-1,2,3-Triazolo[4,5-b]pyridin-3-oxy)tris-1-pyrrolidinyl hexafluorophosphate | 156311-83-0 |
| PyCIU | 1-(Chloro-1-pyrrolidinylmethylene)pyrrolidine hexafluorophosphate | 135540-11-3 |
| CDI | N,N'-Carbonyl Diimidazole | 530-62-1 |
| TsIm | 1-p-toluenesulfonyl imidazole | 2232-08-8 |
| TPSI | 1-(2,4,6-triisopropylphenylsulfonyl)imidazole | 50257-40-4 |
| TSTU | 2-succinimidyl-1,1,3,3-tetramethylurea tetrafluoroborate | 105832-38-0 |
| DEPBT | 3-(diethoxy o-acyloxy)-1,2,3-benzotriazin-4-one | 165534-43-0 |
| DMTMM | 4-(4,6-Dimethoxytriazin-2-yl)-4-methylmorpholine hydrochloride | 3945-69-5 |
| EEDQ | 2-ethoxy-1-ethoxycarbonyl-1,2-dihydroquinoline | 16357-59-8 |
| CIP | 2-chloro-1,3-dimethylimidazolium hexafluorophosphate | 101385-69-7 |
| CIB | 2-chloro-1,3-dimethylimidazolium tetrafluoroborate | 153433-26-2 |
| DMC | 2-chloro-1,3-dimethylimidazolium chloride | 37091-73-9 |
| EDC | 1-(3-Dimethylaminopropyl)-3-ethylcarbodiimide hydrochloride | 7084-11-9 |
| DIC | N,N'-Diisopropylcarbodiimide | 693-13-0 |
| HOAt | N-hydroxy-7-azabenzotriazole | 39968-33-7 |
| HOBt | 1-hydroxybenzotriazole | 2592-95-2 |

### BRIEF DESCRIPTION OF THE DRAWINGS

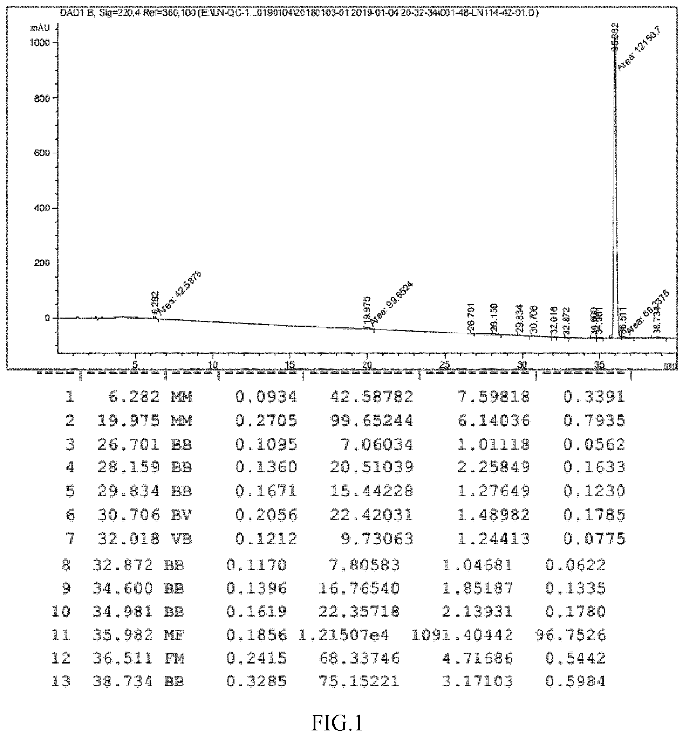

Figure 1 is a high performance liquid chromatography of DMT-3 synthesized in the present disclosure.

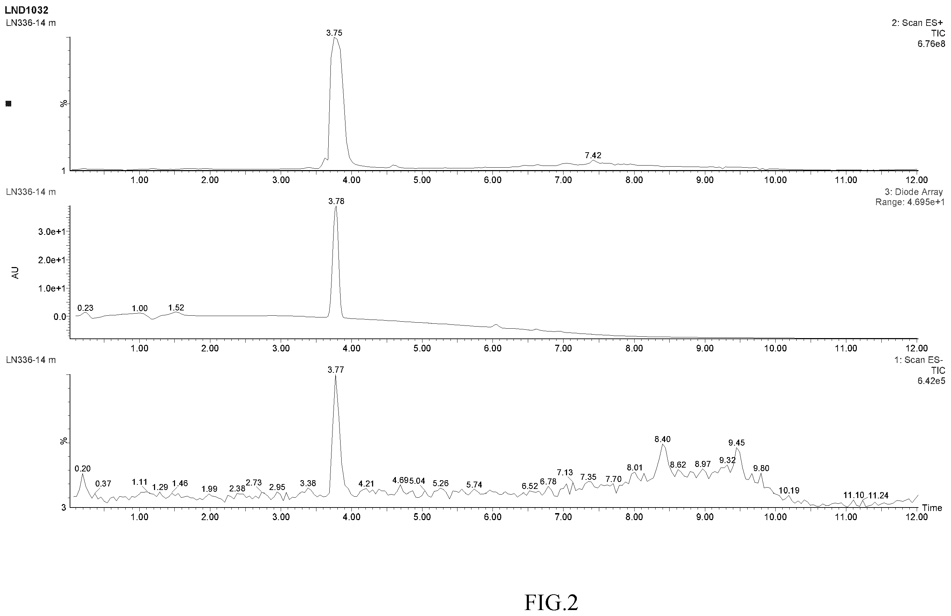

Figure 2 is a liquid chromatography of compound A synthesized in the present disclosure.

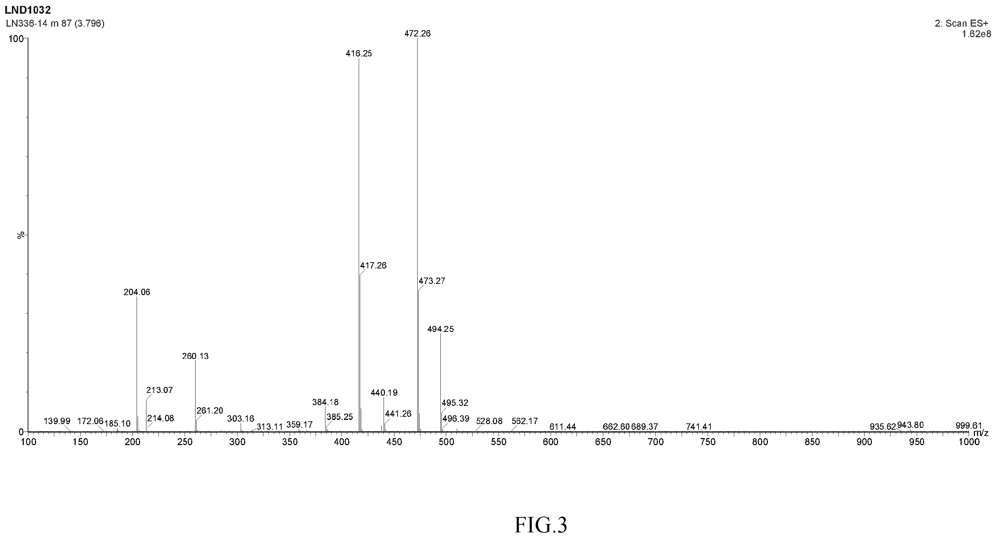

Figure 3 is a mass spectrum of compound A synthesized in the present disclosure.

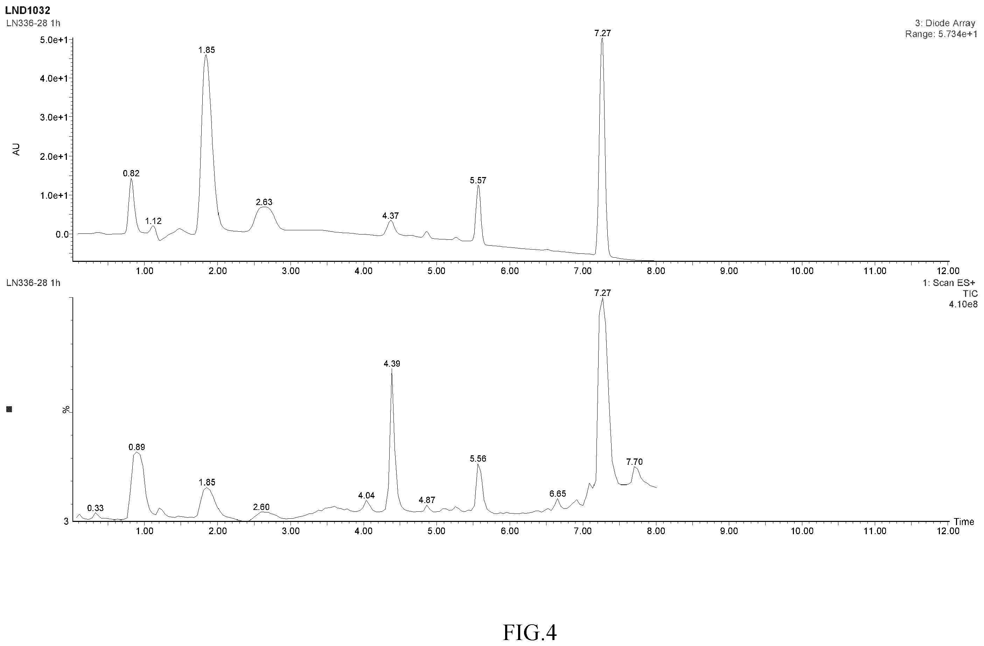

Figure 4 is a liquid chromatography of compound C synthesized in the present disclosure.

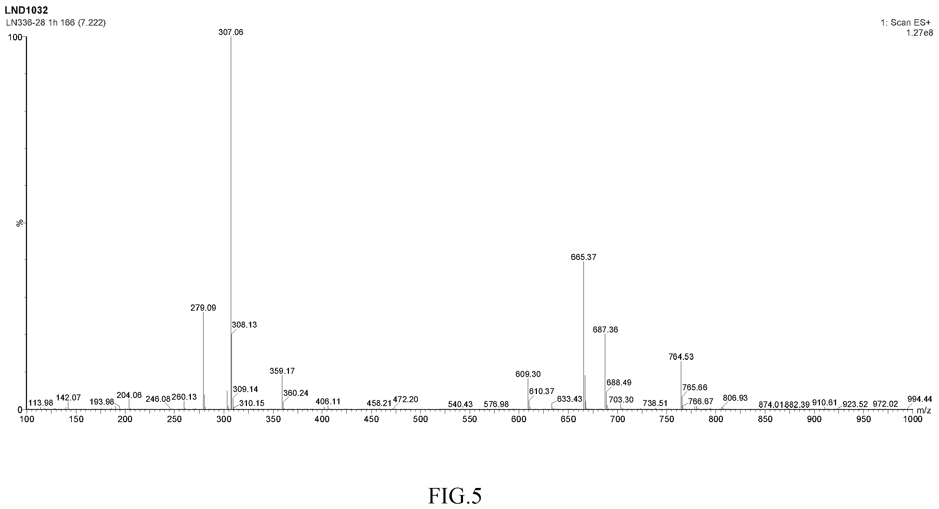

Figure 5 is a mass spectrum of compound C synthesized in the present disclosure.

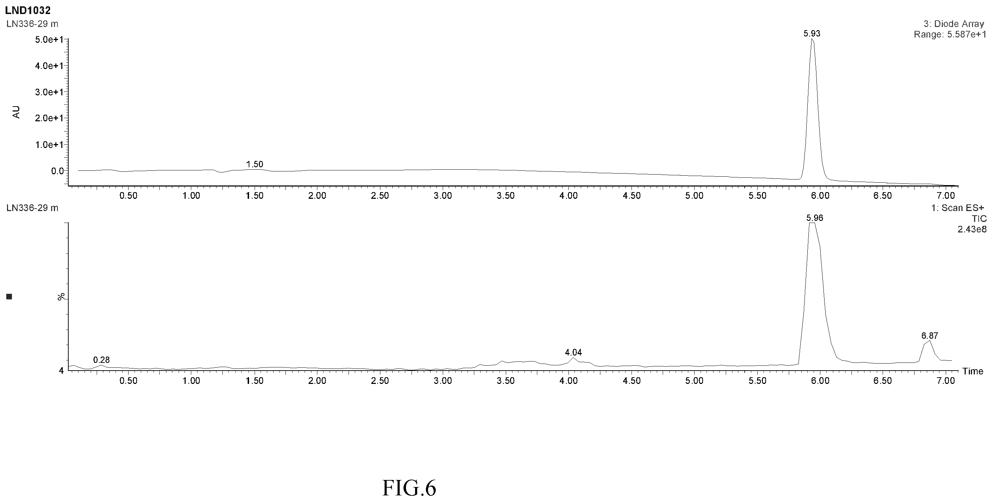

Figure 6 is a liquid chromatography of compound D synthesized by the present disclosure.

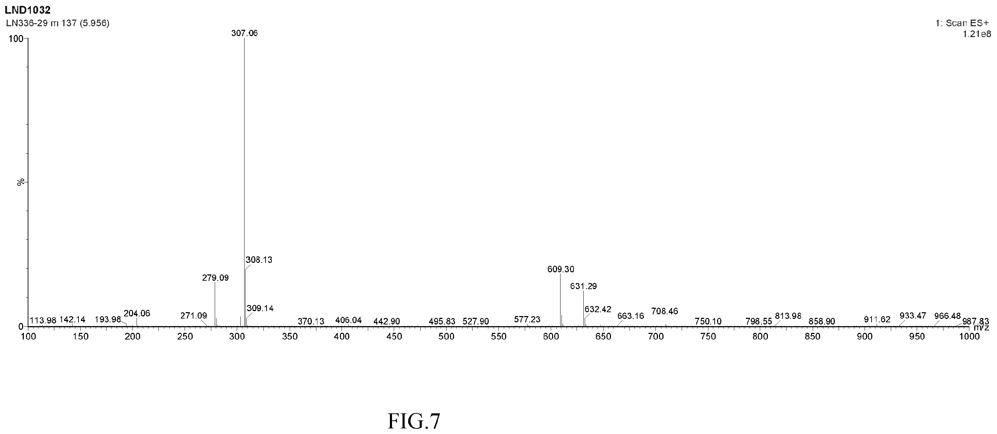

Figure 7 is a mass spectrum of compound D synthesized by the present disclosure.

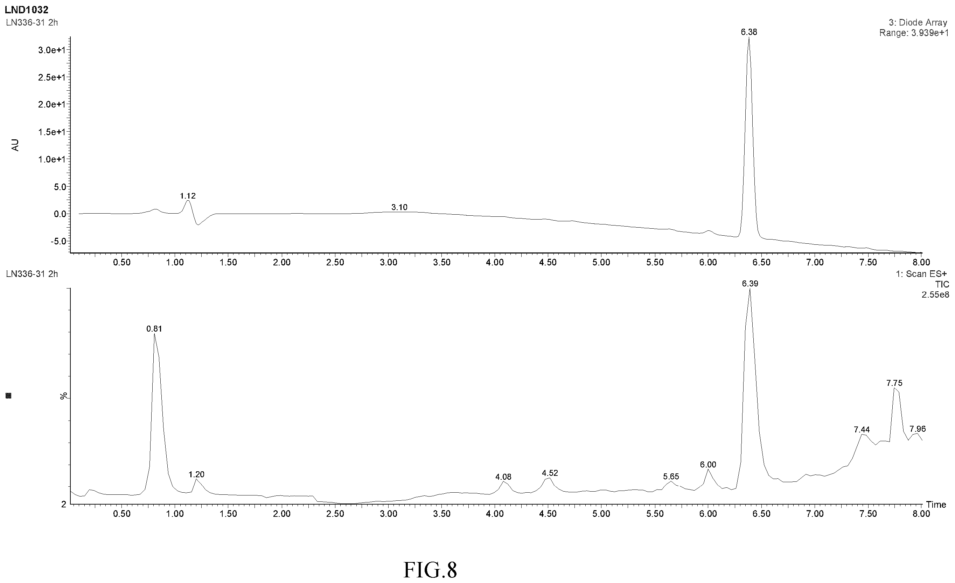

Figure 8 is a liquid chromatography of compound F synthesized in the present disclosure.

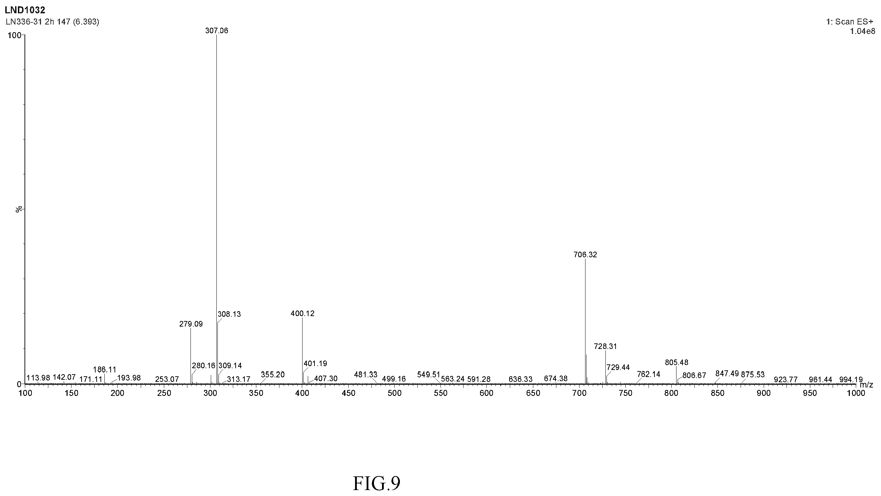

Figure 9 is a mass spectrum of compound F synthesized in the present disclosure.

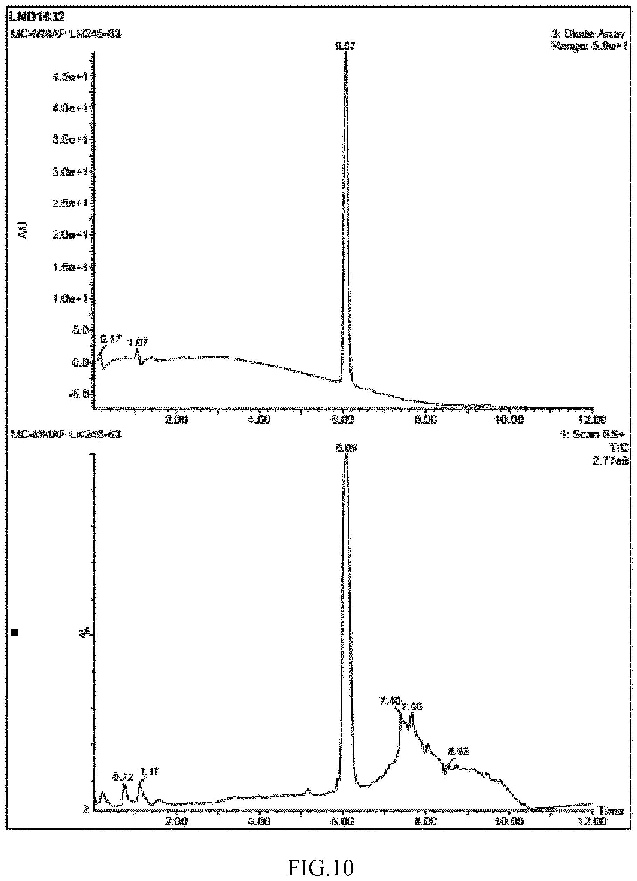

Figure 10 is a liquid chromatography of the target product MC-MMAF synthesized by the present disclosure.

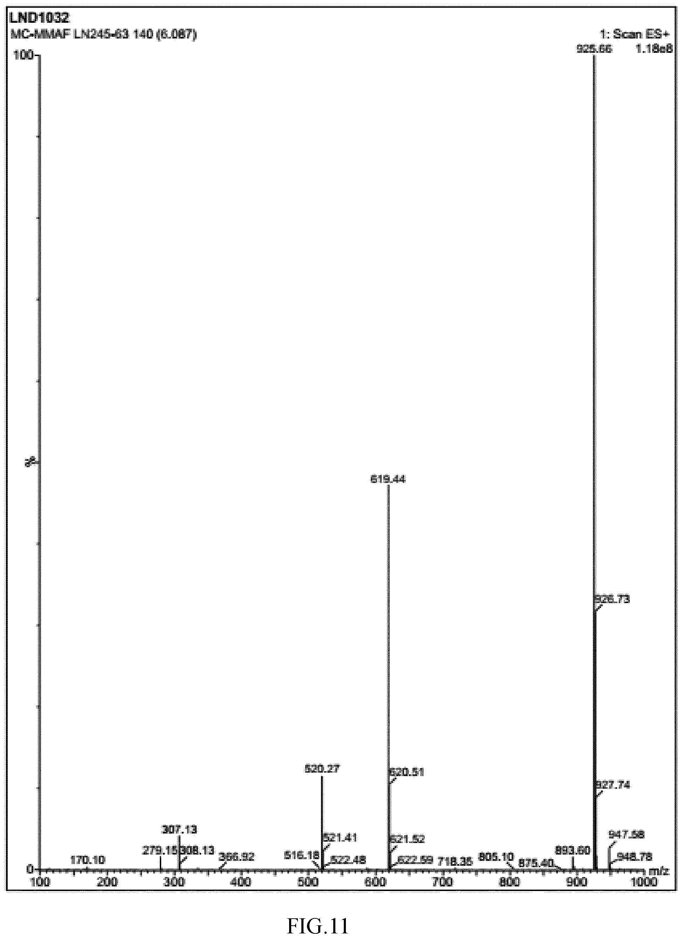

Figure 11 is a mass spectrum of the target product MC-MMAF synthesized by the present disclosure.

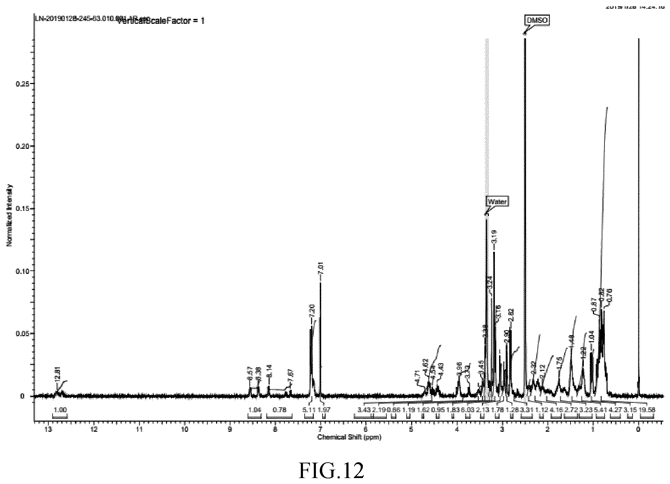

Figure 12 is the NMR spectrum of the target product MC-MMAF synthesized by the present disclosure.

### DESCRIPTION OF THE EMBODIMENTS

LCMS means liquid-mass spectrometry detection method; HPLC means high-performance liquid chromatography detection.

The raw materials and reagents for each step of the reaction involved in the present disclosure can be purchased from the market or prepared according to the method of the present disclosure.

The present disclosure provides a method for synthesizing MC-MMAF, which includes the following steps:
1) Dissolve the compound 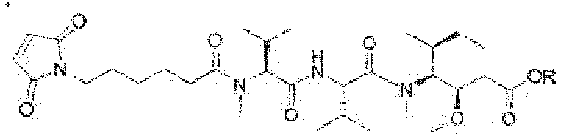 in an appropriate solvent selected from a group consisting of dichloromethane, dimethyl sulfoxide, N,N-dimethylformamide, N,N-dimethylacetamide, tetrahydrofuran, 1,4-dioxane, 2-methyltetrahydrofuran and a mixture thereof, will undergo amide condensation reaction with Dap-Phe-OH to obtain MC-MMAF. The structural formula of Dap-Phe-OH is

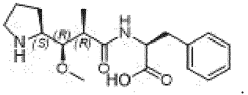

In step 1), if R is hydrogen, in the presence of reagent M, add reagent N, which is selected from a group consisting of DCC, DCEP, EDC, DIC, HATU, HBTU, HBPIPU, HBPyU, HSPyU, HCTU, HOTU , HOTT, HSTU, HDMA, TATU, TBTU, TCTU, TCFH, TDBTU, TOTU, TOTT, TPTU, TFFH, BTFFH, TNTU, TSTU, COMU, T3P, BOP, PyBOP, PyBrOP, PyClOP, Brop, PyAOP, PyCIU, CDI , TPSI, TSTU, DEPBT, DMTMM, EEDQ, CIP, CIB, DMC, HOBt, EDCI and a mixture thereof; more preferably, the reagent M is selected from a group consisting of EDCI, EDC, DIC, HOAt, HOBt and a mixture thereof; further preferably, the reagent M is a mixture of EDCI, EDC, DIC, HOAt and HOBt; most preferably, the reagent M is a mixture of EDCI and HOBt. The reagent N is selected from a group consisting of triethylamine, diisopropylethylamine (DIEA), pyridine, N,N-dimethyl-4-pyridine and a mixture thereof, and is preferably diisopropylethylamine (DIEA). The reaction temperature is 20°C subzero to 40°C, preferably 10°C subzero to 25°C.

In step 1), if R is selected from a group consisting of perimidyl, pentafluorophenyl, p-nitrophenyl, phthalamide and a mixture thereof, in the presence of reagent P, it will interact with Dap-Phe-OH reacts to obtain MC-MMAF. The reagent P is selected from a group consisting of triethylamine, diisopropylethylamine (DIEA), pyridine, N,N-dimethyl-4-pyridine, sodium carbonate, sodium bicarbonate, potassium carbonate, potassium bicarbonate, lithium carbonate, lithium bicarbonate and a mixture thereof, preferably sodium carbonate, or diisopropylethylamine (DIEA). The reaction temperature is 0°C to 100°C, preferably 15°C to 50°C.

In step 1), it also includes the step of separating MC-MMAF from the reaction solution after the reaction is completed. Preferably, the separation includes evaporating the solvent under reduced pressure, and then purifying or recrystallization by medium pressure chromatography to obtain MC-MMAF.

The present disclosure also provides a method of synthesizing 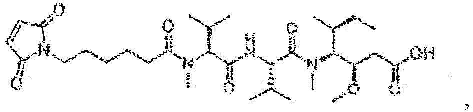 using a polypeptide with a protective group 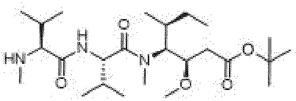 to reaction with 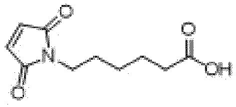 to first synthesize a compound 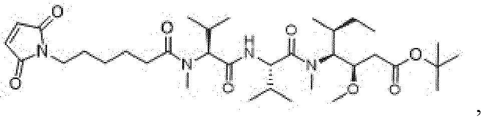 and then deprotect the group under acidic conditions to obtain the compound 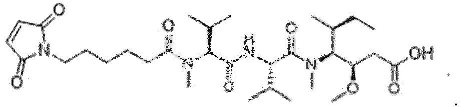 The concentration of trifluoroacetic acid ranges from 30% to 50%, preferably 35%. The deprotection reaction will be completed quickly, and then the reaction will be quenched immediately, and finally the yield of compound D can be increased from 5% to 50%.

### Example 1

The reaction route of this example is as follows:

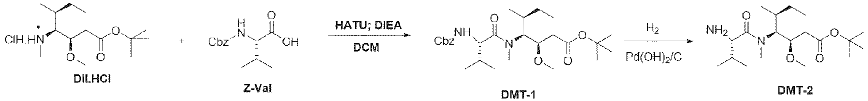

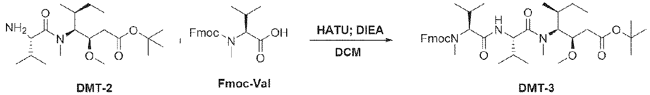

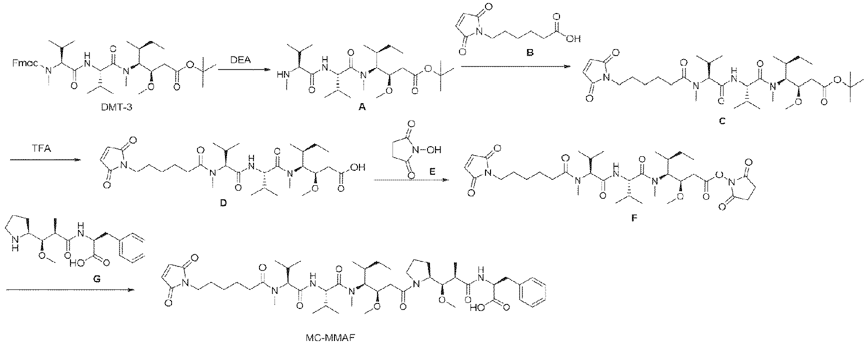

In a 3L three-necked flask, add 1.5L of dichloromethane and Dil.HCl (202.3g, 0.683mol 1.0 eq), magnetic stirring, nitrogen protection, then add Z-Val-OH (163.23g, 0.65mol, 0.95 eq) and HATU (311.6g, 0.82 mol, 1.20 eq), stir at room temperature for 30 minutes, then cool to an ice bath, control the temperature at 10 degree and add DIEA (452.5 ml, 4.0eq) dropwise, after the addition is completed, stir under ice bath for 30 minutes, move to room temperature, react for 16 hours, and detect by HPLC. The main peak is the product peak (retention time 29.98 min). The reaction of the raw material Dil.HC1 is complete and the reaction is complete. Wash the reaction solution with citric acid aqueous solution (2L*1), saturated sodium bicarbonate solution (2L*1), saturated brine (2L*1), dry the organic layer with anhydrous sodium sulfate, filter with suction, desolventize and obtain 531g of crude product. Dissolve the crude product in 800ml methanol, add 1.1ml (1 mol/L) dilute hydrochloric acid (about 1 hour) dropwise with stirring in an ice bath, and stir at room temperature for 12 h. Stop stirring, separate the layers, separate the upper water layer and the lower layer. Dry the products by oil pump, and obtain 325 g DMT-1, and the yield is 91%.

Add 800 ml methanol and DMT-1 (LN114-38,325 g, 0.66 mol) and 110 g Pd(OH)2/C in a 2L single-neck flask, replace with H2 three times, react at room temperature for 5 h, TLC monitors the raw material DMT-1 is complete reacted. Add diatomaceous earth to the sand core funnel, filter with suction, and wash the filter cake with 1L methanol, collect the filtrate, evaporate the filtrate, and pump until the product does not foam, obtain 230.2g of DMT-2, with a purity of 94%; yield: 97 %.

In a 3L three-neck flask, dissolve DMT-2 (LN114-40-01, 230.2g, actual 0.60mol, 1.0 eq) in 500ml DCM, stir well, add Fmoc-Me-val (202.6g, 0.57 mol, 0.95 eq) and HATU (292.9 g, 0.77 mol, 1.20 eq), then add 1L of DCM, stir at room temperature for 30 min, then cool to an ice bath, and add DIEA (212.7 ml, 2.0 eq) dropwise at 10 degree. After stirring under the bath for 30 min, move to room temperature and react for 16.0 h. HPLC detects the main peak as the product peak (retention time 36.00 min). The reaction of the raw material DMT-2 is complete and the reaction is over. Wash the reaction solution with water (2.0 L*1), citric acid aqueous solution (2L*1), saturated sodium bicarbonate solution (2 L*1), saturated brine (1 L*1), and wash the organic layer w with water. After the aqueous sodium sulfate is dried, filter with suction to remove the solvent to obtain the crude product 655g. Dissolve the crude product in 650ml methanol, and add 360ml (1 mol/L) dilute hydrochloric acid dropwise with stirring. Stir at room temperature for 12 h, stop stirring, separate the layers, and separate the upper layer of water, so twice. Dry the lower product with an oil pump to obtain 373 g of DMT-3 with a purity of 96.7% by HPLC and a yield of 90%.

Add compound DMT-3 (5.0 g, 7.22 mmol) and diethylamine (5 mL) to dichloromethane (20 mL), stir and react at room temperature under nitrogen protection for 4 hours. LCMS shows that regard the compound DMT-3 in the reaction solution less than 3 % as the end of the reaction. Spin-dry the reaction solution, and purify the crude product by medium pressure reverse phase (using 220g industrial packed C18 reverse phase column), and purified gradient water/acetonitrile (90/10-10/90, v/v) for 1 hour. Collect the pure product and lyophilize to obtain a white solid compound A (white solid, 3.15 g, yield 93%). MS: 472.26 (M+H⁺).

Add compound B (1.77 g, 8.04 mmol), HATU (3.82 g, 10.05 mmol) and DIEA (1.72 g, 13.4 mmol) to dichloromethane (50 mL), stir and react at room temperature under nitrogen for 30 minutes, and then add the compound A (3.15 g, 6.68 mmol), stir and react at room temperature for 4 hours under the protection of nitrogen, LCMS shows that regard the compound A in the reaction solution less than 3% as the end of the reaction. Wash the reaction solution with citric acid aqueous solution (50mL), saturated brine (50mL), dry with anhydrous sodium sulfate and spin-dry. Purify the crude product by medium pressure reverse phase (use 120g industrial packed C18 reverse phase column), and purified gradient water/Acetonitrile (90/10-10/90, v/v), time 1 hour. Collect the pure product and lyophilize to obtain a white solid compound C (white solid, 3.86 g, yield 87%). MS: 665.37 (M+H⁺)

Dissolve compound C (3.86 g, 5.81 mmol) in a mixed solvent of dichloromethane and trifluoroacetic acid (20 mL, 2/1, v/v), stir and the react at room temperature under nitrogen for 20 minutes. LCMS shows the compound in the reaction solution C is less than 5% as the end of the reaction. Dilute the reaction solution with 40mL acetonitrile, concentrate at low temperature to about 10 mL volume, and purify by medium pressure reverse phase (using 220g industrial packed C18 reverse phase column), and purified gradient water/acetonitrile (90/10-10/90, v/v) ), the time is 2 hours. Collect the pure product and lyophilize to obtain a white solid compound D (1.59 g, yield 45%). MS: 609.30 (M+H⁺)

Dissolve compound D (1.59 g, 2.61 mmol), compound E (0.36 g, 3.13 mmol) and compound EDCI (0.60 g, 3.13 mmol) in dichloromethane (20 mL), and t stir and react at room temperature for 2 hours under nitrogen protection. LCMS shows when the compound D in the reaction solution is less than 5%, the reaction is deemed to be complete. Wash the reaction solution with saturated brine (20mL), dry with anhydrous sodium sulfate, and spin-dry. Purify the crude product by medium-pressure reverse phase (40g industrial packed C18 reverse phase column), and purified gradient water/acetonitrile (90/10-10) /90 ,v/v), the time is 1 hour. Collect the pure product and lyophilize to obtain a white solid compound F (white solid, 1.79 g, yield 97%). MS: 706.32 (M+H⁺)

Dissolve compound F (1.79 g, 2.53 mmol), compound G (0.93 g, 2.78 mmol) and DIEA (0.72 g, 5.56 mmol) in dichloromethane (20mL), stir at room temperature and the reaction is under nitrogen protection for 18 hours. LCMS showed the compound F in the reaction liquid is less than 3%, the reaction is deemed to be complete. Wash the reaction solution with citric acid aqueous solution (20mL) and saturated brine (20mL) successively, dry over anhydrous sodium sulfate, and spin-dry. The crude product is purified by medium pressure reverse phase (Use 80g industrial packed C18 reverse phase column), and pure gradient water /Acetonitrile (90/10-10/90, v/v), time 1 hour. Collect the pure product and lyophilize to obtain a white solid compound MC-MMAF (white solid, 2.01 g, yield 86%, HPLC purity 99% by UV 220nm). MS: 925.66 (M+H⁺)

## Claims

1. An intermediate compound for synthesizing MC-MMAF, its structural formula
is : 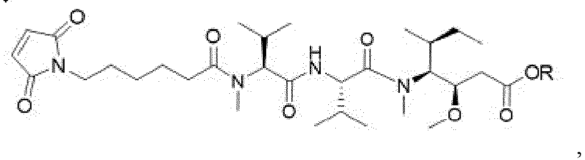 Wherein, R is selected from a group consisting of succinimidyl, p-nitrophenyl, phthalamide and a mixture thereof.

2. A method for synthesizing MC-MMAF, wherein, the method is to perform a condensation reaction on a compound of structural formula 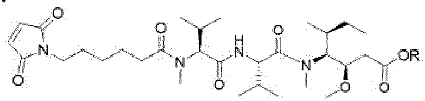 and a compound of structural formula 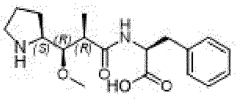 in a solvent,
R is selected from a group consisting of succinimidyl, p-nitrophenyl, and phthalamide, and reacts in the presence of reagent P, which is selected from a group consisting of ethylamine, diisopropylethylamine (DIEA), pyridine, N,N-dimethyl-4-pyridine, sodium carbonate, sodium bicarbonate, potassium carbonate, potassium bicarbonate, lithium carbonate and lithium bicarbonate;
the solvent is selected from a group consisting of dichloromethane, dimethyl sulfoxide, N,N-dimethylformamide, N,N-dimethylacetamide, tetrahydrofuran, 1,4-dioxane and 2-methyl tetrahydrofuran and a mixture thereof;
reaction temperature is 0°C to 100°C.

3. The method according to claim 2, wherein the reagent P is sodium carbonate or diisopropylethylamine (DIEA).

4. The method according to claim 2, wherein the reaction temperature is 15°C to 50°C.

5. The method according to any one of claims 2 to 4, wherein, after the reaction is completed, separate MC-MMAF from the reaction solution.

6. The method according to claim 5, wherein the separation operation comprises evaporating the solvent under reduced pressure, and then purifying or recrystallization by medium pressure chromatography.

## Patentansprüche

1. Zwischenverbindung zur Synthese von MC-MMAF, deren Strukturformel lautet: 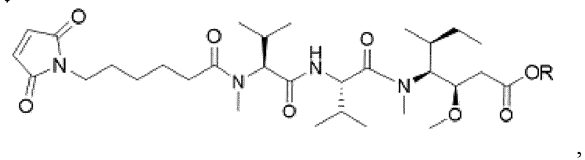 Wobei R ausgewählt ist aus einer Gruppe bestehend aus Succinimidyl, p-Nitrophenyl, Phthalamid und einer Mischung davon.

2. Verfahren zum Synthese von MC-MMAF, wobei das Verfahren darin besteht, eine Kondensationsreaktion an einer Verbindung mit der Strukturformel 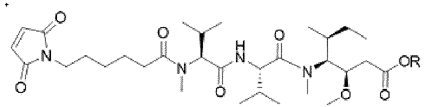 und einer Verbindung mit der Strukturformel 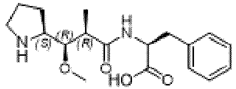 in einem Lösungsmittel durchzuführen,
Wobei R ausgewählt ist aus einer Gruppe bestehend aus Succinimidyl, p-Nitrophenyl und Phthalamid, und reagiert in Gegenwart des Reagenzes P, das ausgewählt ist aus einer Gruppe bestehend aus Ethylamin, Diisopropylethylamin (DIEA), Pyridin, N,N-Dimethyl-4-Pyridin, Natriumcarbonat, Natriumbicarbonat, Kaliumcarbonat, Kaliumbicarbonat, Lithiumcarbonat und Lithiumbicarbonat,
Wobei das Lösungsmittel ausgewählt ist aus einer Gruppe bestehend aus Dichlormethan, Dimethylsulfoxid, N,N-Dimethylformamid, N,N-Dimethylacetamid, Tetrahydrofuran, 1,4-Dioxan und 2-Methyl-Tetrahydrofuran und einer Mischung davon;
Wobei die Reaktionstemperatur 0 °C bis 100 °C beträgt.

3. Verfahren nach Anspruch 2, wobei das Reagenz P Natriumcarbonat oder Diisopropylethylamin (DIEA) ist.

4. Verfahren nach Anspruch 2, wobei die Reaktionstemperatur 15 °C bis 50 °C beträgt.

5. Verfahren nach einem der Ansprüche 2 bis 4, wobei nach Abschluss der Reaktion MC-MMAF von der Reaktionslösung getrennt wird.

6. Verfahren nach Anspruch 5, wobei der Trennvorgang das Verdampfen des Lösungsmittels unter vermindertem Druck und anschließend die Reinigung oder Umkristallisation durch Mitteldruckchromatographie umfasst.

## Revendications

1. Composé intermédiaire pour la synthèse de MC-MMAF, dont la formule développée 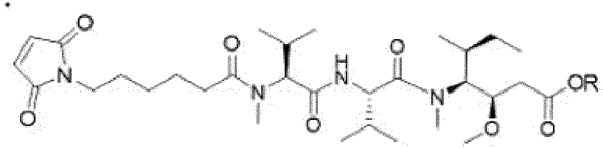 est la suivante : ,
dans lequel R est choisi dans un groupe constitué de succinimidyle, de p-nitrophényle, de phtalamide et d'un mélange de ceux-ci.

2. Procédé de synthèse de MC-MMAF, dans lequel le procédé consiste à effectuer une réaction de condensation sur un composé ayant la formule développée 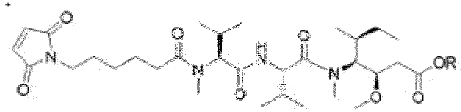 et un composé ayant la formule développée 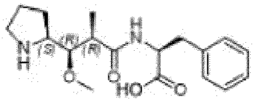 dans un solvant,
R est choisi dans un groupe constitué de succinimidyle, de p-nitrophényle et de phtalamide, et réagit en présence d'un réactif P, qui est choisi dans un groupe constitué d'éthylamine, de diisopropyléthylamine (DIEA), de pyridine, de N,N-diméthyl-4-pyridine, de carbonate de sodium, de bicarbonate de sodium, de carbonate de potassium, de bicarbonate de potassium, de carbonate de lithium et de bicarbonate de lithium ;
le solvant est choisi dans un groupe constitué de dichlorométhane, de diméthylsulfoxyde, de N,N-diméthylformamide, de N,N-diméthylacétamide, de tétrahydrofurane, de 1,4-dioxane et de 2-méthyl tétrahydrofurane et d'un mélange de ceux-ci ;
la température de réaction est de 0 °C à 100 °C.

3. Procédé selon la revendication 2, dans lequel le réactif P est le carbonate de sodium ou la diisopropyléthylamine (DIEA).

4. Procédé selon la revendication 2, dans lequel la température de réaction est de 15 °C à 50 °C.

5. Procédé selon l'une quelconque des revendications 2 à 4, dans lequel, une fois la réaction terminée, le MC-MMAF est séparé de la solution de réaction.

6. Procédé selon la revendication 5, dans lequel l'opération de séparation comprend l'évaporation du solvant sous pression réduite, puis la purification ou la recristallisation par chromatographie à pression moyenne.
